Europäisches Patentamt

European Patent Office

Office européen des brevets

(11) Publication number: **0 300 988 A2**

# EUROPEAN PATENT APPLICATION

(21) Application number: **88870125.7**

(22) Date of filing: **22.07.88**

(51) Int. Cl.4: **C 07 K 15/12**
**A 61 K 37/02**

(30) Priority: **23.07.87 US 77366**

(43) Date of publication of application:
**25.01.89 Bulletin 89/04**

(84) Designated Contracting States:
**AT BE CH DE ES FR GB GR IT LI LU NL SE**

(71) Applicant: **WASHINGTON UNIVERSITY**
**Lindell and Skinker Boulevards**
**St. Louis Missouri 63130 (US)**

(72) Inventor: **Broze, George John, Jr.**
**15 Westpoint Lane**
**St. Louis Missouri 63131 (US)**

(74) Representative: **Lunt, John Cooper et al**
**Monsanto Europe S.A. Patent Department Avenue de**
**Tervuren 270-272 Letter Box No 1**
**B-1150 Brussels (BE)**

(54) **Tissue factor inhibitor.**

(57) A purified tissue factor inhibitor is provided which has the following characteristics:

A. it exists in two forms, $TFI_1$ migrating at about 37-40,000 daltons and $TFI_2$ at about 25-26,000 daltons, as determined by sodium dodecylsulfate polyacrylamide gel electrophoresis,

B. it has a partial N-terminal amino acid sequence as follows:

```
1                                                    15
X-X-Glu-Glu-Asp-Glu-Glu-His-Thr-Ile-Ile-Thr-Asp-Thr-Glu-

16                                          27
Leu-Pro-Pro-Leu-Lys-Leu-Met-His-Ser-Phe-(Phe)-Ala
```

wherein X-X has not been determined, and

C. it exhibits inhibitory activity to Factor $X_a$.

EP 0 300 988 A2

**Description**

## TISSUE FACTOR INHIBITOR

### Background of the Invention

This invention relates to a coagulation inhibitor known as tissue factor inhibitor (TFI).

The coagulation cascade that occurs in mammalian blood comprises two distinct systems - the so-called intrinsic and extrinsic systems. The latter system is activated by exposure of blood to tissue thromboplastin (Factor III), hereinafter referred to as tissue factor (TF). Tissue factor is a lipoprotein that arises in the plasma membrane of many cell types and in which the brain and lung are particularly rich. Upon coming into contact with TF, plasma Factor VII or its activated form, Factor $VII_a$, forms a calcium-dependent complex with TF and then proteolytically activates Factor X to Factor $X_a$, and Factor IX to Factor $IX_a$.

Early studies concerning the regulation of Tf-initiated coagulation showed that incubation of TF (in crude tissue thromboplastin preparations) with serum inhibited its activity in vitro and prevented its lethal effect when it was infused into mice. Extensive studies by Hjort, Scand. J. Clin. Lab. Invest. 9, Suppl. 27, 76-97 (1957), confirmed and extended previous work in the area, and led to the conclusion that an inhibitory moiety in serum recognized the Factor VII-TF complex. Consistent with this hypothesis are the facts that the inhibition of TF that occurs in plasma requires the presence of $Ca^{2+}$ (which is also necessary for the binding of Factor $VII/VII_a$ to TF) and that inhibition can be prevented and/or reversed by chelation of divalent cations with EDTA. More recent investigations by the present inventor and others have shown that not only Factor $VII_a$ but also catalytically active Factor $X_a$ and an additional factor are required for the generation of TF inhibition in plasma or serum. See Broze and Miletich, Blood 69, 150-155 (1987), and Sanders et al., Ibid., 66, 204-212 (1985). This additional factor, defined herein as tissue factor inhibitor (TFI), is present in barium-absorbed plasma and appears to be associated with lipoproteins, since TFI functional activity segregates with the lipoprotein fraction that floats when serum is centrifuged at a density of 1.21 $g/cm^3$. According to Broze and Miletich, supra, HepG2 cells (a human hepatoma cell line) secrete an inhibitory moiety with the same characteristics as the TFI present in plasma.

### Brief Description of the Invention

In accordance with the present invention, a novel tissue factor inhibitor (TFI) is provided which has the following characteristics:

A. it exists in two forms, $TFI_1$ migrating at about 37-40,000 daltons and $TFI_2$ at about 25-26,000 daltons, as determined by sodium dodecylsulfate polyacrylamide gel electrophoresis (SDS PAGE),

B. it has a partial N-terminal amino acid sequence as follows:

```
1                                                    15
X-X-Glu-Glu-Asp-Glu-Glu-His-Thr-Ile-Ile-Thr-Asp-Thr-Glu-

16                                                   27
Leu-Pro-Pro-Leu-Lys-Leu-Met-His-Ser-Phe-(Phe)-Ala
```

wherein X-X has not been determined, and

C. it exhibits inhibitory activity to Factor $X_a$.

The novel TFI of this invention has been isolated in highly purified forms in which it did not exist in the cell and tissue sources from which it has been obtained. That is, it has been prepared in purified forms which are essentially free of other proteins, and free from other cellular components and tissue matter.

The novel TFI exhibits biological activity which suggests that it is important to medical science in the study of the coagulation cascade. In particular, the novel TFI of this invention has indicated therapeutic use as a coagulation inhibitor and anti-thrombotic agent.

In a preferred embodiment, the novel TFI of this invention has been secreted by HepG2 cells grown in nutrient culture medium and then isolated from the conditioned medium. The isolation is preferably carried out in an essentially three step method as follows:

A. $CdCl_2$ precipitation,

B. Factor $X_a$ Affi-Gel 15 affinity chromatography of the resolubilized precipitate, and

C. Superose 12 gel filtration of the fractions from the affinity chromatography containing TFI activity.

In an alternate method of isolation, the Factor $X_a$ in step B is inactivated by binding to diisopropylphosphate

(iPr$_2$P), Sephadex G-75 is used as the gel filtration material in step C, and a fourth step D is carried out as follows:

D. Mono Q ion-exchange chromatography of the fractions from the gel filtration containing TFI activity.

In the foregoing methods, HepG2 is a well-known and widely available human hepatoma cell line whose establishment and characteristics are described in U.S. Patent 4,393,133. Samples of this cell line also are available from the permanent collection of the American Type Culture Collection, Rockville, Maryland, under acession number ATCC HB 8065.

Other suitable cell sources of the TFI are, for example, SK-HEP-1 cells (ATCC HTB 52), Chang Liver cells (ATCC CCL 13), endothelial cells and mammalian blood sera.

The HepG2 cells can be grown at about 37°C in conventional nutrient culture medium to express the TFI. A preferred medium is Earle's modified essential medium (EMEM), which is commercially available. Other suitable commercially available media are described, for example, by Helen J. Morton, In Vitro 6(2), 89-108 (1970). These nutrient culture media contain amino acids, minerals, carbohydrates, vitamins and are frequently fortified with mammalian sera, e.g., fetal bovine sera or calf sera, and various growth factors. As used herein, the HepG2 cells use preferably first grown in sera-containing medium and then transferred to sera-free medium supplemented with growth stimulating amounts of transferrin, selenium, insulin, liver cell growth factor and lactalbumin hydrolysate.

The CdCl$_2$ precipitate formed in step A, above, is preferably diluted with an equal volume of a buffer solution prior to application to the affinity chromatography column in step B. The buffer solution should comprise an aqueous solution of a biologically acceptable buffer material adjusted to a pH of about 7.5. Use of 0.05M Tris-HCl is illustrative. An essentially similar buffer can then used to equilibrate the affinity chromatography column.

The Affi-Gel® 15 used in step B is a commercially available, activated affinity support prepared as a N-hydroxysuccinimide ester of a derivatized cross-linked agarose gel.

The Superose® 12 and Sephadex® G-75 used in the gel filtration step C are commercially available, bead-formed gels prepared by cross-linking dextran with epichlorohydrin. The preferred superfine grades have a diameter on the order of about 20-50 µM.

Prior to application to the gel filtration column, the fractions from the affinity chromatography containing TFI activity in step B are preferably concentrated such as by ultrafiltration to reduce the amount of working solution, and the column can be equilibrated with 1M NaSCN, buffered to pH of about 7.5.

In the alternate isolation method, following the gel filtration step C, the fractions containing the TFI activity are preferably precipitated with acetone, and the precipitate is then resolubilized for application to the Mono Q column. The latter column can be equilibrated with 6M urea, buffered to pH of about 8.3.

The Mono Q used in step D is a commercially available, strong anion exchange resin based on a hydrophilic polymer in bead form, diameter about 10 µM, with quaternary amino charged groups.

Although particular methods of isolating the TFI are described herein, it will be understood that the novel TFI is not limited to any specific method of preparation. Thus, the TFI protein can be made by conventional recombinant DNA technology. Recent advances in biochemistry and in recombinant DNA technology have made it possible to synthesize specific proteins, for example, enzymes, under controlled conditions independent of the organism from which they are normally isolated. These biochemical synthetic methods employ enzymes and subcellular components of the protein synthesizing systems of living cells, either in vitro in cell-free systems, or in vivo in microorganisms. In either case, the principal element is provision of a deoxyribonucleic acid (DNA) of specific sequence which contains the information required to specify the desired amino acid sequence. Such a specific DNA sequence is termed a gene. The coding relationships whereby a deoxyribonucleotide sequence is used to specify the amino acid sequence of a protein is well-known and operates according to a fundamental set of principles. See, for example, Watson, Molecular Biology of the Gene, 3d ed., Benjamin-Cummings, Menlo Park, Calif., 1976.

A cloned gene may be used to specify the amino acid sequence of proteins synthesized by in vitro systems. RNA-directed protein synthesizing systems are well-established in the art. Double-stranded DNA can be induced to generate messenger RNA (mRNA) in vitro with subsequent high fidelity translation of the RNA sequence into protein.

It is now possible to isolate specific genes or portions thereof from higher organisms, such as man and animals, and to transfer the genes or fragments to microorganisms such as bacteria or yeasts. The transferred gene is replicated and propogated as the transformed microorganism replicates. Consequently, the transformed microorganism is endowed with the capacity to make the desired protein or gene which it encodes, for example, an enzyme, and then passes on this capability to its progeny. See, for example, Cohen and Boyer, U.S. Pats. 4,237,224 and 4,468,464.


Detailed Description of the Invention


While the specification concludes with claims particularly pointing out and distinctly claiming the subject matter regarded as forming the present invention, it is believed that the invention will be better understood from the following description of preferred embodiments taken in conjunction with the accompanying drawings in which briefly:

FIG. 1 is a graphical representation which shows the elution profile following iPr$_2$P- human Factor X$_a$ Affi-Gel affinity chromatography of a concentrated TFI preparation isolated from HepG2 cells in one embodiment of the invention.

FIG. 2 is a graphical representation which shows the elution profile following Sephadex G-75 gel filtration of a concentrated sample from the TFI active fractions of FIG. I.

FIG. 3 is a graphical representation which shows the elution profile following Mono Q ion-exchange chromatography of a concentrated sample from the TFI active fractions of FIG. 2.

FIG. 4 shows the SDS PAGE of a purified TFI sample from FIG. 3.

FIG. 5 is a graphical representation which shows a comparison of purified TFI from FIG. 3 and normal human serum in a TFI assay.

FIG. 6 is a graphical representation which shows the inhibitory effect of the purified TFI from FIG. 3 on Factor X$_a$ compared to its lack of effect on thrombin.

FIG. 7 is a graphical representation which shows the rapid inhibition of TF activity by the purified TFI from FIG. 3 in the presence of Factor VII$_a$, Factor X$_a$ and Ca$^{++}$.

FIG. 8 is a graphical representation which shows the elution profile following bovine Factor X$_a$ Affi-Gel affinity chromatography of a concentrated TFI preparation isolated from HepG2 cells in another embodiment of the invention.

FIG. 9 is a graphical representation which shows the elution profile following Superose 12 gel filtration of a concentrated sample from the TFI active fractions of FIG. 8.

FIG. 10 shows the SDS PAGE of a reduced, purified TFI sample from FIG. 9.

FIG. 11 shows the SDS PAGE of an unreduced, purified TFI sample from FIG. 9.

FIG. 12(A) shows the SDS PAGE of TFI isolated from Chang Liver cells (lane 2), SK-HEP-1 (lanes 3 and 4), and HepG2 cells (lanes 5 and 6) by immunoaffinity chromatography in further embodiments of the invention, with standard molecular weight markers shown in lane 1.

FIG. 12(B) shows the Western Blotting (electrophoretic transfer) on nitrocellulose paper and staining with $^{125}$I-X$_a$ followed by autoradiography of electrophoretically separated proteins from lanes 2, 3, 4 and 6 of FIG. 12(A) in lanes 1, 2, 3 and 4, respectively.

In order to illustrate specific preferred embodiments of the invention in greater detail, the following exemplary laboratory preparative work was carried out. In Example 1 TFI is isolated by the four step method and in Example 2 by the three step method from conditioned media of HepG2 cells as defined hereinbefore. In Example 3 TFI is isolated from several cell sources by immunoaffinity chromatography.

## EXAMPLE 1

Materials. Affi-Gel 15 and low molecular weight standards for polyacrylamide electrophoresis were purchased from Bio-Rad Laboratories. Na$^{125}$I (carrier-free) was purchased from New England Nuclear, and Iodo-Gen was obtained from Pierce Chemical Co. Sephadex G-75 superfine and a Mono Q column were received from Pharmacia Inc. Earle's modified essential medium (EMEM) and fetal bovine and calf sera were obtained from KC Biological, Inc. (Lenexa, KS), and liver cell growth factor was obtained from Miles Laboratories. Bovine serum albumin, phenylmethylsulfonyl fluoride, diiospropyl fluorophosphate (iPr$_2$P-F), acrylamide, methylenebis(acrylamide), rabbit brain cephalin, transferrin, selenium, insulin, lactalbumin hydrolysate, HEPES (N-2-hydroxyethylpiperazine-N'-2-ethanesulfonic acid), MOPS (3-[N-morpholino]propanesulfonic acid), and Trizma base [Tris(hydroxymethyl)aminoethane], were supplied by Sigma Chemical Co. All other chemicals were of reagent grade or better and came from Fisher Scientific Co. or from Sigma. Factor X-deficient human plasma was obtained from George King Biomedical (Overland Park, KS). Serum samples from healthy blood donors were provided by the American Red Cross (St. Louis, MO). HepG2 cells were obtained from the American Type Culture Collection.

Proteins. A crude prepration of TF was prepared and washed extensively with EDTA [Broze and Miletich, Blood 69, 150-155 (1987); Broze and Majerus, J. Biol. Chem. 255, 1242-1247(1980)]. The Factor X coagulant protein from Russell's viper venom (XCP), antithrombin IIIa, Factor VII$_a$, and human Factor X were purified as described by Broze and Miletich, supra; Broze et al., J. Biol. Chem. 260, 10917 (1985); Peterson and Blackburn, J. Biol. Chem. 260, 610-615(1985); and Miletich, Broze and Majerus, Methods Enzymol. 80, 221-228 (1981). Factor X$_a$ was produced from purified Factor X by incubation with insolubilized X coagulant protein and inactivated with iPr$_2$P-F [Broze and Miletich, supra; Bajaj et al., Prep. Biochem. 11, 397-412 (1981)]. iPr$_2$P-Factor X$_a$ was linked to Affi-Gel 15 at a final concentration of ≈2 mg/ml of packed gel, using the manufacturer's published instructions (MOPS buffer, pH 7.5).

Assay. A three-stage assay for TF inhibition was used during the purification procedure, below. [Broze and Miletich, supra]. In the first stage, 10 μl of Factor VII$_a$ (1 μg/ml), 10 μl of Factor X (10 μg/ml), 10 μl of CaCl$_2$ (40 mM), 10 μl of antithrombin IIIα (650 μg/ml), 50 μl of the sample to be tested, diluted in TBSA buffer (0.1 M NaCl/0.5 M Tris-HCl, pH 7.5, containing bovine serum albumin at 1 mg/ml), and 10 μl of crude, EDTA-washed TF (10% vol/vol) were incubated at room temperature. After 30 min. a 10 μl sample was diluted 100-fold into TBSA buffer with 5 mM CaCl$_2$. Fifty microliters of this diluted sample, 50 μl of Factor VII$_a$ (1 μg/ml), 50 μl of

CaCl$_2$ (25 mM), and 50 μl of Factor X (10 μg/ml) were then incubated at 37°C. After 1 min., 50 μl of a mixture containing 10 parts Factor X-deficient plasma and 1 part rabbit brain cephalin stock reagent (prepared as described by Bell and Altone, Nature 174, 880(1954) and reconstituted according to the published instructions of the supplier, Sigma) was added, and the time to clot formation was determined with a fibrometer (Baltimore Biological Laboratory, Cockeysville, MD).

mixtures in which TBSA rather than sample was incubated in the first stage served as controls. The concentration of crude TF in the assay was chosen to produce control clotting times of 35-40 sec. Antithrombin IIIα was included in the assay to decrease the effect of the Factor X$_a$ formed during the first-stage incubation upon the clotting time derived in the third stage of the assay. Relative TFI activity was calculated from a standard curve constructed by plotting (on log-log paper) the prolongation in seconds of the clotting time beyond the control value vs. the final concentration of normal pooled serum (50 donors) in the first stage of the assay. This standard curve produced a linear response from 1-10% (vol/vol) serum concentrations. One unit of TFI activity was defined as that contained in 1 ml of normal pooled serum. Results of assays of chromatography fractions are expressed as clotting times and have not been converted to units/ml.

NaDodSO$_4$/PAGE was performed in 15% gels (4% stacking gel) by the method of Laemmli, Nature(London)227, 680-685 (1970). Reduced samples were heated to 100°C for 5 min. in the presence of 10% 2-mercaptoethanol prior to electrophoresis. TFI was extracted following NaDodSO$_4$/PAGE by cutting the lane from the gel, allowing it to soak in 0.1 M NaCl/0.05 M Tris-HCl, pH 7.5, for 30 min., slicing it into 2-mm sections and incubating each slice overnight in 100 μl of 1 M NaSCN/0.05 M Tris-HCl, pH 7.5/0.025% Lubrol PX (a biological nonionic detergent consisting of an ethylene oxide condensate of fatty alcohol) containing bovine serum albumin at 0.5 mg/ml. A 1:50 dilution in TBSA of the extracted material was then assayed as described above for TF inhibition. Western blotting was performed as described by Broze, Hickman and Miletich, J. Clin. Invest. 76, 937-946 (1985), using $^{125}$I-labeled Factor X$_a$ as the probe. The amino acid composition of purified TFI (110 pmol) was determined, following 24-hr hydrolysis in 6 M HCl at 110°C, on a Beckman 6300 autoanalyzer with postcolumn ninhydrin derivatization.

Cell Culture. HepG2 cells were cultured in plastic or glass roller bottles (850 cm$^2$). After initial seeding, the cells were grown in EMEM with nonessential amino acids and penicillin (10$^3$ units/ml), streptomycin (100 μg/ml), amphotericin (250 ng/ml), sodium pyruvate (100 mM), L-glutamine (200 mM), and 5% fetal bovine serum and 5% calf serum, with twice weekly medium changes. After 2 weeks, the medium containing serum was removed, and the cells were washed gently with EMEM and thereafter cultured in serum-free medium consisting of the same ingredients listed above plus transferin (5 μg/ml), selenium (5 Pg/ml), insulin (5 μg/ml), liver cell growth factor (20 ng/ml), lactoalbumin hydrolysate (5 mg/ml), and HEPES (25 mM). The conditioned medium was removed and replaced with fresh serum-free medium twice per week. The HepG2 cells can be maintained under these conditions for >3 months.

N-Terminal Amino Acid Sequence Determination. Automated Edman degradation chemistry was used to determine the NH$_2$-terminal protein sequence of the purified TFI. An Applied Biosystems, Inc., model 470A gas phase sequencer (Foster City, CA) was employed for the degradations, Hunkapiller et al., Methods Enzymol. 91, 399-413 (1983). The respective PTH-amino acid derivatives were identified by RP-HPLC analysis in an on-line fashion employing an Applied Biosystems, Inc., Model 120A PTH Analyzer fitted with a Brownlee 2.1 mm I.D. PTH-C$_{18}$ column. Electroblotting technology was applied to the sample in order to sequence bands isolated directly from SDS-PAGE. The methods employed were as described by Aebersold et al., J. Biol. Chem. 261, 4229-4238 (1986). Using these sequencing methods, the first two N-terminal amino acids could not be established, but amino acids 3-27 were determined as follows:

```
1                                               15
X-X-Glu-Glu-Asp-Glu-Glu-His-Thr-Ile-Ile-Thr-Asp-Thr-Glu-


                                              27
Leu-Pro-Pro-Leu-Lys-Leu-Met-His-Ser-Phe-(Phe)-Ala
```

Purification of TFI. Except as otherwise noted the purification procedure was performed at room temperature. The purification method is summarized in an essentially four step scheme in Table 1, below.

Table 1. Purification of TFI

| Step | Protein mg* | Activity, units | Specific activity, units/mg | Yield, % | Purification, fold |
|---|---|---|---|---|---|
| Serum-free medium | 23,400 (2840)+ | 4400 | 0.88(1.55)± | 100 | 1 |
| $CdCl_2$ precipitation, elution, and dialysis | 480 | 4090 | 8.52 | 93 | 45 (5.50)± |
| $iPr_2P$-Factor $X_a$-Affi-Gel | 7.0 | 1240 | 180 | 28 | 957 (116)± |
| Sephadex G-75 | 0.83 | 870 | 1050 | 20 | 5,590 (677)± |
| Mono Q | 0.06 | 590 | 9800 | 13 | 52,100 (6320)± |

*Calculated assuming an extinction coefficient at 280 nm of $E_{1cm}^{1\%} = 10$

+Medium extensively dialyzed against TS buffer.

±Values based upon dialyzed medium with $M_r$ 10,000 cut-off.

The detailed purification procedure was as follows.

A. <u>Cadmium chloride precipitation</u>. HepG2 cell serum-free conditioned medium (4 liters), following the addition of phenylmethylsulfonyl fluoride (final concentration 0.1 mM) and $NaN_3$ (final concentration 0.05% wt/vol) was centrifuged at 2500 x g for 30 min. to remove particulate debris. $CdCl_2$ (1.0 M) was then added to a final concentration of 5 mM, and the mixture was stirred for 15 min. The precipitate, which contained the TFI activity, was collected by centrifugation (2500 x g for 30 min.), and the supernatant was decanted. The pellet was dissolved with 40 ml of 0.5 M EDTA/5 mM $iPr_2P$-F, pH 9.5, and then dialyzed extensively against TS buffer (0.1 M NaCl/0.05 M Tris-HCl/0.1 mM phenylsulfonyl fluoride/0.5 mM EDTA/0.02% $NaN_3$, pH 7.5).

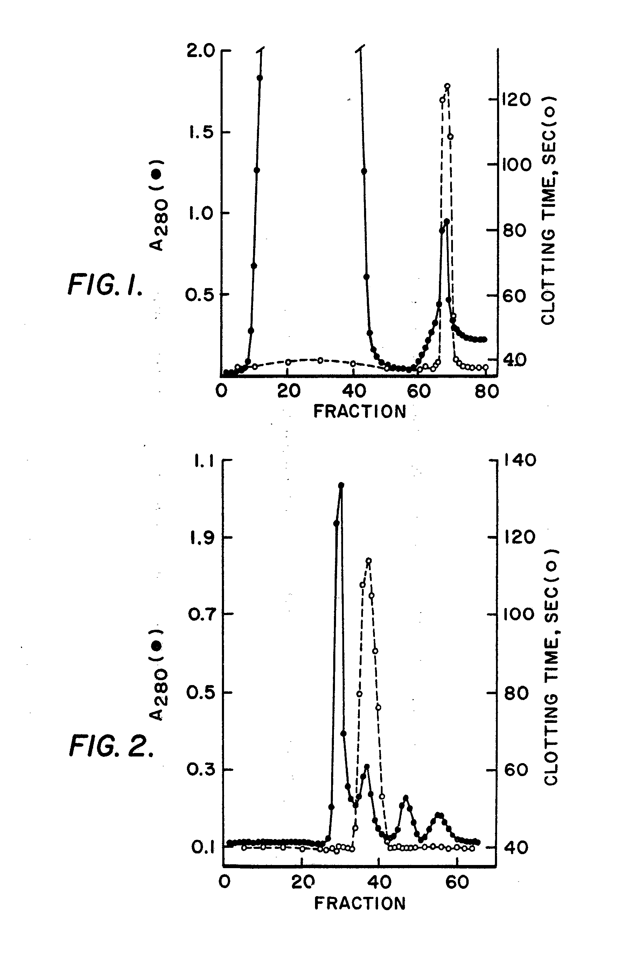

B. $iPR_2$-Factor $X_a$ Affi-Gel 15 Chromatography. The dialyzed preparation was clarified by centrifugation (10,000 x g for 15 min.) and applied to a column of $iPr_2P$-Factor $X_a$-Affi-Gel 15 equilibrated in TS buffer. The gel was washed with starting buffer, and bound material was eluted with 2 M NaSCN/0.05 M Tris-HCl, pH 7.5/0.05% Lubrol PX/0.02% $NaN_3$/0.1 mM phenylmethylsulfonyl fluoride (FIG. 1). Fractions containing TFI activity were pooled and concentrated to ≈ 1 ml, using a YM5 hydrophilic ultrafiltration membrane (Amicon).

C. <u>Gel filtration and acetone delipidation</u>. The concentrated sample was applied to a column of Sephadex G-75 superfine equilibrated in 1 M NaSCN/0.05 M Tris-HCl, pH 7.5/0.05% Lubrol PX/0.02% $NaN_3$/0.1 mM phenylmethylsulfonyl fluoride (FIG. 2). Fractions containing TFI activity were pooled and mixed with 8 volumes of acetone. After 30 min., the precipitate was collected by centrifugation (10,000 x g, 20 min. 10°C).

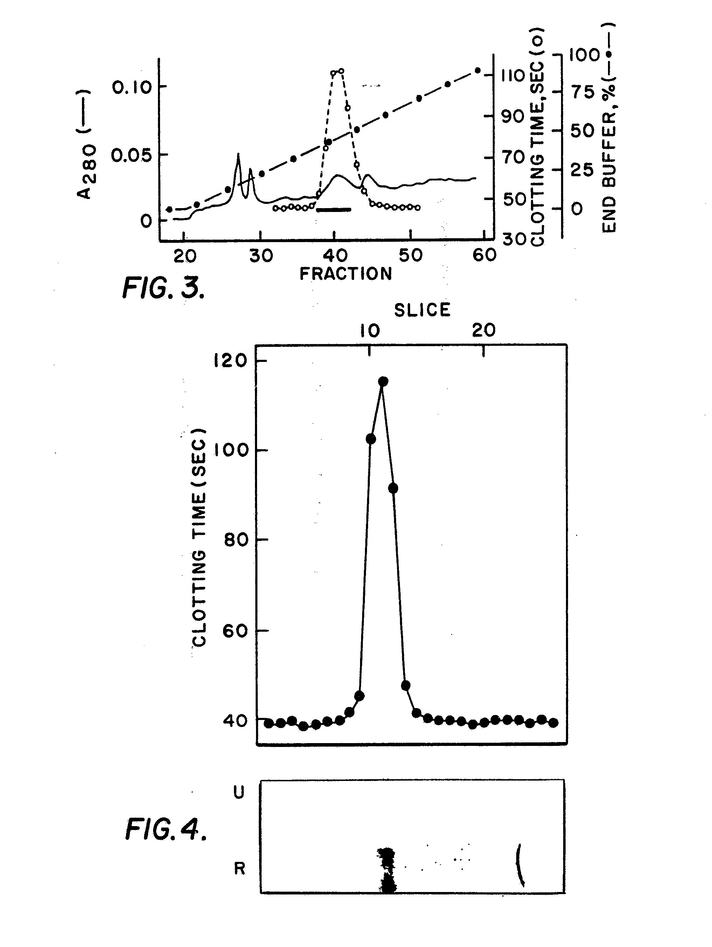

D. <u>Mono Q ion-exchange chromatography</u>. The acetone precipitate was solubilized with 1.0 ml of 6 M urea/0.02 M Tris-HCl/0.05% Lubrol PX, pH 8.3, and applied to a Mono Q column equilibrated in the same buffer. The column was developed with a 30-ml linear gradient from starting buffer to 6 M urea/0.5 M Tris-HCl/0.05% Lubrol PX, pH 8.3 (FIG. 3). Fractions containing TFI activity were pooled as indicated in FIG. 3, concentrated to ≈ 1 ml (YM5, Amicon), dialyzed against 1 M NaSCN/0.05 M Tris-HCl/0.05% Lubrol PX, pH 7.5, and stored at 4°C.

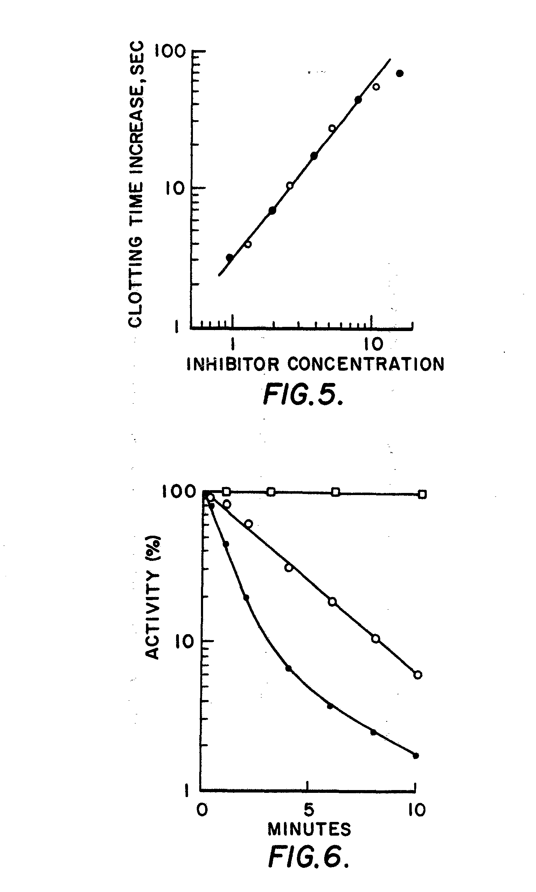

<u>Properties of the TFI</u>. $NaDodSO_4$/PAGE of the purified TFI showed a predominant band at $M_r$ 38,000 (unreduced) or 39,000 (reduced) (FIG. 4). Extraction of gel slices following $NaDodSO_4$/PAGE of an unreduced sample of the purified preparation showed that inhibitory activity comigrated with the Coomassie-stained band. As expected, this same band was recognized by [125]I-labeled factor $X_a$ upon immunoblotting (data not shown). The amino acid composition of the purified TFI is shown in Table 2, below. The purified TFI appeared to be similar to the inhibitor present in serum in that it required Factor $VII_a$, Factor X, and $Ca^{2+}$ for the expression of activity [Broze and Miletich, supra] (Table 3, below), and dilutions of the purified TFI produced a line parallel to that of the normal human serum standard curve (FIG. 5).

Incubation of the purified TFI (250 ng/ml) with Factor $X_a$ (25 ng/ml) led to a loss of $X_a$ coagulation activity in a functional bioassay (FIG. 6). This inhibitory effect of TFI was not a general property directed against all serine proteases since thrombin was completely unaffected by incubation with TFI (FIG. 6).

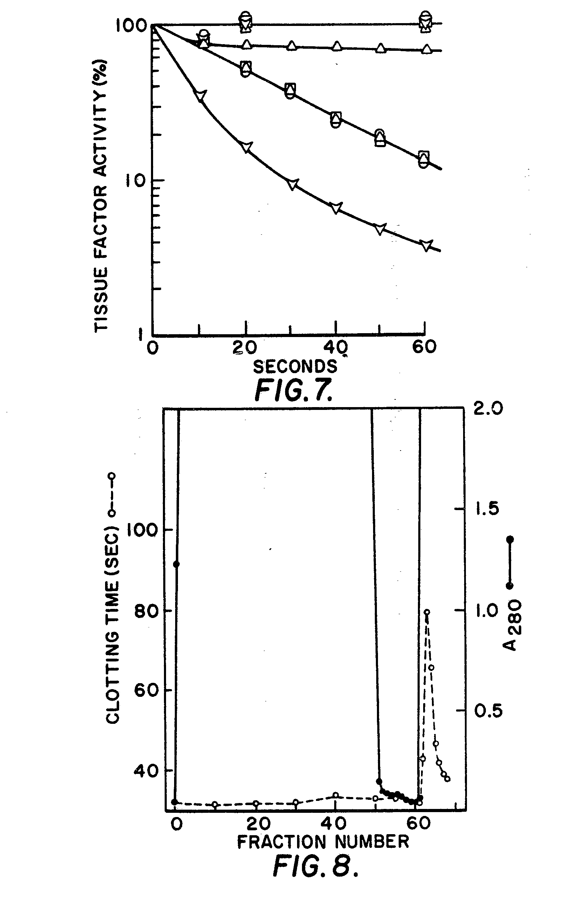

In the presence of Factor $VII_a$, Factor $X_a$ and $Ca^{2+}$, TFI produced very rapid inhibition of TF activity with an apparent $t_{1/2}$ of 20 seconds (FIG. 7). The inclusion of heparin (0.5 units/ml) in the mixtures accelerated the initial rate of inhibition 3 fold ($t_{1/2}$ = 6 seconds). Inhibition of apparent TF activity by TFI was not affected by the inclusion of antithrombin $III\alpha$ (65 μg/ml) in the reaction.

Table 2. Amino acid composition of TFI

| Amino acid | Abbrev. | Mol per 38,000 g of protein |
|---|---|---|
| Aspartic acid | Asp | 40 |
| Threonine | Thr | 20 |
| Serine | Ser | 19 |
| Glutamic acid | Glu | 44 |
| Proline | Pro | 15 |
| Glycine | Gly | 33 |
| Alanine | Ala | 19 |
| Cysteine | Cys | 13* |
| Valine | Val | 12 |
| Methionine | Met | + |
| Isoleucine | Ile | 15 |
| Leucine | Leu | 27 |
| Tyrosine | Tyr | 10 |
| Phenylalanine | Phe | 20 |
| Histidine | His | 5 |
| Lysine | Lys | 26 |
| Arginine | Arg | 18 |
| Tryptophan | Trp | ND |

ND, not determined.
*Cysteine value from 24-hr hydrolysis; performic acid oxidation was not performed.
+Methionine was oxidized during hydrolysis; none was detected.

0 300 988

Table 3. Requirement for Factor VII, Factor X, and $Ca^{2+}$ for the expression of activity by purified TFI

| Deletion from first stage* | Clotting time,+ sec |
|---|---|
| None | 95.3 |
| Factor X | 40.2 |
| Factor VII$_a$ | 41.3 |
| $Ca^{2+}$ (5 mM EDTA) | 40.8 |
| Control | 37.7 |

*See description of TFI assay, above.
+Results are the means of duplicate assays. The final concentration of TFI was 10 ng/ml in the first stage of the assay.

The functional properties of the TFI isolated from HepG2 conditioned medium, above, were illustrated as follows.

Coagulation Assays: Factor $X_a$ inhibition by TFI. Reaction mixtures containing purified TFI (250 ng/ml), Factor $X_a$ (25 ng/ml) and either EDTA (1 mM) or CaCl$_2$ (5 mM) and a 1:40 dilution of stock rabbit brain cephalin (prepared according to the published instructions of Sigma) in TBSA (0.1 M NaCl, 0.05 M Tris-HCl, pH 7.5, 1 mg/ml bovine serum albumin) were incubated at room temperature. At various times, a 100 $\mu$l subsample was added to a fibrometer cup already containing 50 $\mu$L of CaCl$_2$ (25 mM) and 50 $\mu$L of rabbit brain cephalin (1 to 10 dilution of stock) at 37°. Fifty $\mu$L of Factor X deficient plasma was then added immediately and the time to clot formation determined on a fibrometer (BBL, Cockeysville, MD). Factor $X_a$ activity was determined by comparing the clotting times to a standard curve constructed using dilutions of $X_a$ in the same incubation mixtures lacking TFI.

Thrombin Inhibition by TFI: Reaction mixtures containing TFI (400 ng/ml) and thrombin (0.5 units/ml, 180 ng/ml) were incubated at room temperature in TBSA. At various time points a 100 $\mu$L subsample was added to 100 $\mu$L of 0.15 M NaCl, 6.6 gm/L PEG-6000, 10 mM Imidazole, 10 mM CaCl$_2$, pH 7.4, warmed to 37°. Fifty $\mu$L of fibrinogen (2 mg/ml) was then added immediately and the time to clot formation determined on a fibrometer. Relative thrombin activity was determined using a standard curve constructed with dilutions of thrombin.

Inhibition of TF by TFI: One hundred $\mu$L of a mixture containing Factor VII$_a$ (200 ng/ml), Factor $X_a$ (200 ng/ml), CaCl$_2$ (8 mM), and TF (2% v/v), which had been incubated at room temperature for 1 minute, was added to 100 $\mu$L of TBSA containing TFI (600 ng/ml) with or without antithrombin III$\alpha$ (130$\mu$g/ml), and heparin (1 unit/ml). At specified time points thereafter a 10 $\mu$L sample was removed and diluted 100 fold into TBSA with 5 mM CaCl$_2$. Due to practical considerations, the diluted samples made from earlier time points were held until the final, 1 minute, sample had been obtained, and then all were assayed for residual TF activity using a two stage assay. Fifty $\mu$L Factor VII$_a$ (1 $\mu$g/ml), 50 $\mu$L CaCl$_2$ (25 mM), 50 $\mu$L diluted sample, and 50 $\mu$L Factor X (10 $\mu$g/ml) were incubated at 37°. After 1 minute, 50 $\mu$L of a mixture containing 10 parts Factor X deficient plasma and 1 part rabbit brain cephalin stock reagent (prepared as described by Sigma) was added and the time to clot formation was determined with a fibrometer. Since this TF assay involves a dilution of sample from the original incubation mixture and a further 1-2 minute incubation, the derived inhibition rates are considered herein as "apparent" rates.

The results of the above laboratory preparative work leading to the isolation and functional testing of the purified TFI are further exemplified by the following detailed description of FIGS. 1 to 7 of the drawings.

FIG. 1. This figure shows the iPr$_2$P-Factor $X_a$-Affi-Gel affinity chromatography. The dialyzed TFI preparation ($\approx$ 100 ml) following CdCl$_2$ precipitation and extraction was applied to a 1.5 x 40-cm column of iPr$_2$P-Factor $X_a$-Affi-Gel 15 equilibrated in 0.1 M NaCl/0.05 M Tris-HCl, pH 7.5/0.5 mM EDTA/0.02% NaN$_3$/0.1 mM phenylmethylsulfonyl fluoride. After washing with starting buffer, the TFI was eluted with 2 M NaSCN/0.01 mM Tris-HCl, pH 7.5/0.05% Lubrol PX/0.1 mM phenylmethylsulfonyl fluoride. The flow rate was 3 ml/hr, and fraction size was 4 ml. Samples were diluted 500-fold for TFI assay. Fractions 66-70 were

9

pooled.

FIG. 2. This figure shows the Sephadex G-75 superfine gel filtration. The concentrated sample ($\approx$ 1 ml) from iPr$_2$P-Factor X$_a$-Affi-Gel chromatography was applied to a 1 x 120-cm column of Sephadex G-75 superfine. The flow rate was 1.5 ml/hr, and fraction size was 1 ml. Samples were diluted 2000-fold for TFI assay. Fractions 34-41 were pooled.

FIG. 3. This figure shows the Mono Q ion-exchange chromatography. After acetone precipitation, TFI was solubilized with 6 M urea/0.02 M Tris-HCl, pH 8.3/0.05% Lubrol PX and applied to a 1-ml Mono Q column equilibrated in the same buffer. After washing with starting buffer, the column was developed with a 30 ml gradient from starting buffer to ending buffer (6 M urea/0.5 M Tris-HCl, pH 8.3, 0.05% Lubrol PX). The flow rate was 20 ml/hr, and fraction size was 1 ml. Samples were diluted 2000-fold for TFI assay. Horizontal bar indicates fractions that were pooled.

FIG. 4. This figure shows the NaDodSO$_4$/PAGE of purified TFI. Purified TFI (3.75 $\mu$g per lane) was subjected to NaDodSO$_4$/PAGE either unreduced (2 lanes) or following reduction with 10% 2-mercaptoethanol (1 lane). (Upper) One of the lanes containing unreduced TFI was cut from the gel and sliced, and TFI activity was extracted for assay. (Lower) Coomassie blue staining of reduced (R) and unreduced (U) TFI. Origin is at left.

FIG. 5. This figure shows a comparison of purified TFI and normal human serum in TFI assay. Dilutions of purified TFI were assayed in the TFI functional assay and compared to a standard curve constructed using normal human serum. The y axis is prolongation of the clotting beyond the control value (39 sec.), and the x axis is the final concentration of TFI ($\bullet$, in ng/ml) or normal human serum (o in % vol/vol) in the first stage of the assay.

FIG. 6. This figure shows the effect of TFI upon Factor X$_a$ and thrombin. Reaction mixtures containing purified TFI and either X$_a$ or thrombin were constructed as described above. At specified times, samples were removed and assayed for residual enzyme activity by bioassay. $\square$--$\square$, thrombin; $\bullet$--$\bullet$, X$_a$; O--O, X$_a$ with phospholipids and Ca$^{++}$. In control mixtures lacking TFI there was no loss of X$_a$ or thrombin activity (not shown).

FIG. 7. This figure shows the inhibition of Tissue Factor (TF) by TFI. Mixtures containing VII$_a$ (100 ng/ml), X$_a$ (100 ng/ml), Ca$^{++}$ (4 mM), TF (1% v/v), TFI (300 ng/ml) with or without antithrombin III$\alpha$ (65 $\mu$g/ml) and heparin (0.5 units/ml) were constructed at room temperature. At the specified times, samples were removed, diluted, and assayed for remaining TF activity. o--o, without antithrombin III$\alpha$ or heparin; $\nabla$--$\nabla$, with heparin but without antithrombin III$\alpha$; $\square$--$\square$, with antithrombin III$\alpha$ but without heparin; $\triangle$--$\triangle$, with antithrombin III$\alpha$ and heparin; open symbols on the top horizontal line (100% activity) represent the respective incubation mixtures in the absence of TFI.

## EXAMPLE 2

isolation of TFI from the conditioned medium of HepG2 cells was carried out as in Example 1, above, except with variations as follows:

1. The isolation was carried out in an essentially three step method instead of the four step method as defined hereinbefore.

2. In the CdCl$_2$ precipitation step A, a different buffer system (see below) was employed and the dialysis was omitted.

3. The Factor X$_a$ Affi-Gel 15 affinity chromatography in step B utilized bovine Factor X$_a$ without binding to iPr$_2$P instead of the human Factor X$_a$ bound to IPr$_2$P.

4. Superose 12 gel was used instead of Sephadex G-75 gel in the gel filtration step C.

Detailed conditions of these variations were as follows:

### CdCl$_2$ Precipitation

HepG2 cell serum-free conditioned media (4 liters) was centrifuged at 2,500 x g for 30 minutes to remove particulate debris. CdCl$_2$ (1.0 M) was then added to a final concentration of 10 mMM, and the mixture was stirred for 30 minutes. The precipitate, which contained the TFI activity, was collected by centrifugation (10,000 x g for 20 minutes) and the supernatant decanted. The pellet was dissolved in 80 ml of 0.5 M EDTA, 100 KI units/ml aprotonin (Sigma), pH 9.5, and insoluble material removed by centrifugation (10,000 x g for 20 minutes).

### Bovine Factor X Purification

Bovine Factor X was purified from the barium sulfate eluate of bovine plasma (Sigma Chemical Co., St. Louis). 40 units (each from 1 liter of plasma) of eluate were resuspended in 1.6 liters of H$_2$O and the pH adjusted to 6.0 with HCl. Dry benzamidine was added to a final concentration of 1 mM and the mixture centrifuged at 10,000 x g and 4°C to remove insoluble material. The sample was then applied (100 ml/hr) to a 5 x 95 cm column of DEAE Sepharose which had been equilibrated in 0.02 M sodium citrate, I mM benzamidine,

0.02% NaN$_3$, pH 6.0, at 4°. After washing the column with 2 liters of starting buffer, it was developed with a 15 liter gradient from starting buffer to 0.8 M NaCl, 0.02 M sodium citrate, 1 mM benzamidine, 0.02% NaN$_3$, pH 6.0. Fractions containing bovine Factor X$_1$, and X$_2$, which eluted after Protein C but before Protein Z, were pooled, concentrated to - 40 ml (PM10, Amicon, Lexington, MA), and dialyzed into 0.1 M MOPS, pH 7.5. The yield was 68 mg of purified Factor X. Factor X$_a$ was produced by activation of Factor X with insolubilized XCP (Factor X coagulant protein from Russell's viper venom).

Bovine Factor X$_a$ Affi-Gel Affinity Chromatography

The sample was diluted with an equal volume of 0.1 M NaCl, 0.05 M Tris-HCl, pH 7.5, 0.2% Lubrol PX and applied at a flow rate of 4 ml/hr to a 2 ml column of Sepharose 4 B agarose connected in series with a 3 ml column of bovine Factor X$_a$-Affi-Gel which had been equilibrated in 0.1 M NaCl, 0.05 M Tris-HCl, pH 7.5, 0.1% Lubrol PX. The columns were washed with 0.1 M NaCl, 0.05 M Tris-HCl, pH 7.5, 0.1% Lubrol (20 ml), and then 0.1 M NaCl, 0.05 M Tris-HCl, pH 7.5, 2% Lubrol PX (20 ml). The Sepharose 4 B pre-column was then removed from the circuit and the bovine Factor X$_a$-Affi Gel column was further washed with 0.1 M NaCl, 0.05 M Tris-HCl, pH 7.5, 2% β-Octylglucoside (20 ml) and eluted with 0.5 M benzamidine, 0.05 M Tris-HCl, pH 7.5, 2% β-Octyl-glucoside. Fractions containing TFI as determined by the TFI assay were pooled (FIG. 8, Fractions 62-66) and concentrated to ~ 0.4 using a YM5 membrane (Amicon Corp., Danvers, Mass.). The large quantity of A$_{280}$ in the eluted fractions is due to benzamidine.

Superose 12 Gel Filtration

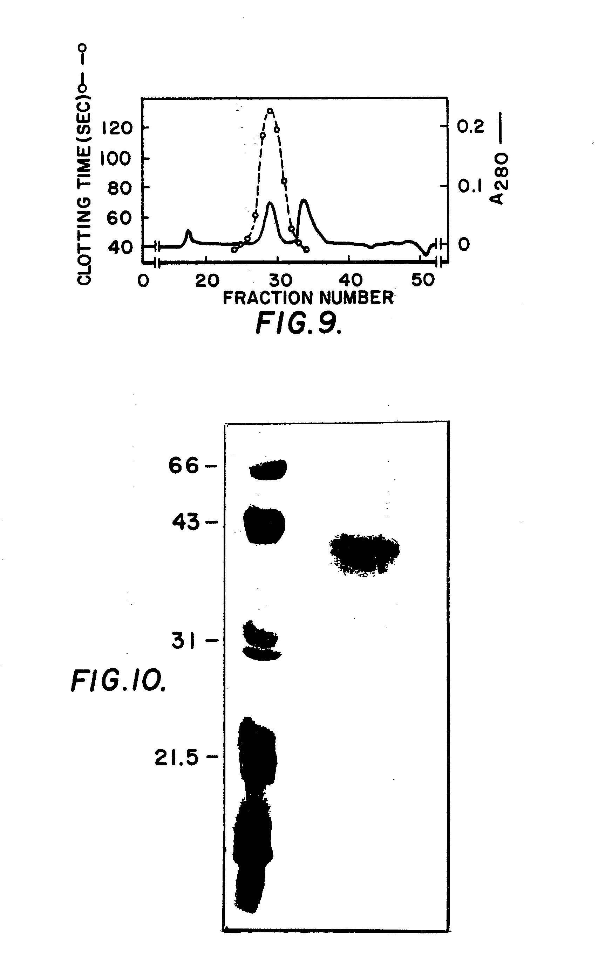

The concentrated sample ( ~ 0.4 ml) was applied to two 25 ml Superose 12 columns connected in series which were equilibrated in 1 M NaSCN, 2% β-Octylglucoside, 0.02 M MOPS, pH 7.0. The columns were developed with starting buffer at a flow rate of 0.3 ml/min. Fractions 27-31 containing TFI activity were identified and pooled (FIG. 9).

Characterization of TFI

SDS-PAGE - Sodium dodecyl sulfate polyacrylamide gel electrophoresis of the reduced purified TFI material revealed a diffuse band of an apparent molecular weight of 39,000 daltons when stained with silver nitrate. Elution of gel slices showed that functional TFI activity comigrated with the protein band (FIG. 10).

Western Blot and Staining with $^{125}$I-X$_a$

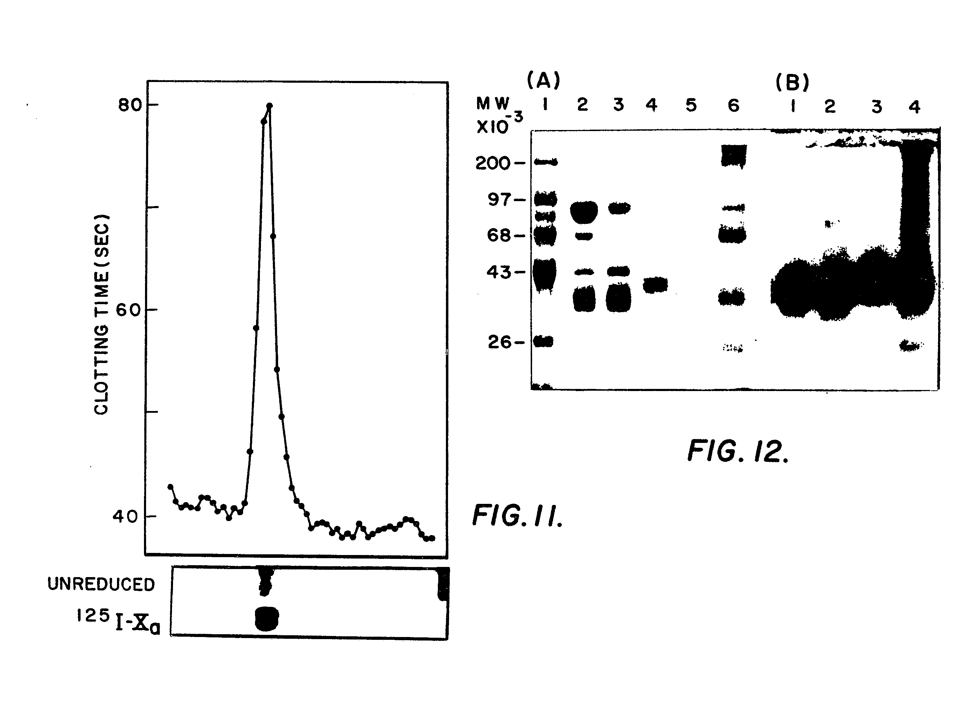

The purified material binds Factor X$_a$. The TFI pool from Superose 12 chromatography was subjected to SDS-PAGE and electrophoretically transferred to nitrocellulose. Subsequent incubation of the nitrocellulose with $^{125}$I-X$_a$ showed that the purified protein binds Factor X$_a$ (FIG. 11).

The results of the above laboratory preparative work in Example 2 leading to the isolation of purified TFI are further exemplified by the following detailed description of FIGS. 8 to 11 of the drawings.

FIG. 8. This figure shows the bovine Factor X$_a$ - Affi-Gel affinity chromotography. Fractions 1 to 60 were 4 ml in volume, and 60 to 70, 1 ml in volume. Absorbance (A$_{280}$) is shown as ●--●; TFI activity of 1000-fold diluted samples o--o.

FIG. 9. This figure shows the Superose 12 gel filtration. Fractions 27-31 containing TFI activity were pooled and stored at -4°C. Absorbance (A$_{280}$) is shown as a solid line --; TFI activity of 1000-fold diluted sample is shown as o--.

FIG. 10 shows the SDS PAGE of reduced, purified TFI. The purified TFI (2 μg) was subjected to NaDodSO$_4$/PAGE following reduction with 10% 2-mercaptoethanol and silver staining. Molecular weight markers in kilodaltons are shown in the left lane. The right lane shows a diffuse band of the reduced TFI with an apparent molecular weight of 39,000 daltons.

FIG. 11 shows the SDS PAGE of unreduced, purified TFI on the upper blot whereby the purified TFI (2 μg) was subjected to NaDOdSO$_4$/PAGE and silver staining. The lower $^{125}$I-X$_a$ blot shows the unreduced TFI (1 μg) electrophoretically transferred to nitrocellulose, stained with $^{125}$I-X$_a$ and audoradiographed. The upper panel of the figure shows the TFI activity of extracted cell slices following SDS PAGE of 1 μg of TFI.

EXAMPLE 3

Isolation of TFI from several cell sources, namely Chang Liver cells, SK-HEP-1 cells, and HepG2 cells was carried out by immunoaffinity chromatography using as the immunogen a synthetic peptide having an amino acid sequence 3-25 of the mature TFI described hereinbefore as follows:

11

## Immunization

A TFI-peptide containing a sequence corresponding to the amino acid sequence 3-25 of the mature TFI was synthesized using Biosystem's solid phase peptide synthesis. The TFI-peptide (5 mg) was conjugated to 10 mg of Keyhole lympet hemocyanin by glutaraldehyde. Two New Zealand white rabbits were each immunized by intradermal injection with a homogenate containing 1 ml of Freund complete adjuvant and 1 ml of the conjugate (200 µg of TFI-peptide). One month later,, the rabbits were each boosted with a homogenate containing 1 ml of Freund incomplete adjuvant and 1 ml of the conjugate (100 µg of TFI-peptide). Antiserum was collected each week thereafter. Booster injection was performed monthly until the rabbits were exsanguinated after 3 months.

## Isolation of anti-TFI-peptide-Ig

The synthetic TFI-peptide (3 mg) was coupled to 0.8 g of CNBr-activated Sepharose® 4B using the manufacturer's published procedure (Pharmacia). To isolate specific antibody, pooled antiserum (15 ml) was mixed with equal volume of a solution (PNBT) containing PBS, 0.4M NaCl, 0.1 M benzamidine and 1% Triton® X-100 and chromatographed on the TFI-peptide Sepharose 4B column at room temperature. The column was washed with 30 ml of PNBT solution and then with the same solution without Triton X-100. The bound antibody was eluted with 0.1 M glycine/HCl, pH 2.2, immediately neutralized by adding 1/10 volume of 1 M Tris-OH and extensively dialyzed against saline solution. Approximately 6.5 mg anti-TFI-peptide Ig was isolated from 15 ml of antiserum.

## Cell Culture

Chang liver, SK hepatoma and HepG2 cells were grown to confluency in Dulbecco's Modified Eagle's Medium (DMEM) supplemented with 10% fetal bovine serum, 50 units/ml penicillin and 50 µg/ml streptomycin in 175-cm$^2$ flasks. Five flasks each of cells were trypsinized and used to seed one 10-chamber cell factory (Nunc). After confluency ( ~ 1 week), the cells were washed with PBS two times and incubated in a serum free medium. The serum free medium consisted of DMEM supplemented with 0.5% lactalbumin hydrolysate, 50 units/ml aprotinin, ITS premix (insulin-transferin-selenium, Collaborative Research product), 20 ng/ml liver cell growth factor (glycyl-histidyl-lysine) and 100 ng/ml phorbol 12-myristate 13-acetate. The serum free medium was replaced with fresh medium every 3 days. The cells can be maintained under these conditions for > 2 months. The pooled conditioned media were made 0.02% NaN$_3$ and 0.01% Triton X-100 and stored at 4°. Some media were concentrated 20 to 100 fold by ultrafiltration using Amicon's YM30 spiral membrane system.

## Immunoaffinity purification of TFI

Isolated anti-TFI-peptide-Ig (20 mg) was coupled to 2 g of CNBR-activated Sepharose 4B by the manufacturer's published procedure. The bed volume of the gel was 7 ml. To isolated TFI, the conditioned media (unconcentrated or concentrated) from Chang liver, SK hepatoma or HepG2 cells were chromatographed on the anti-TFI-peptide-Ig Sepharose 4B column at a speed of 2 ml/min in the cold room, until significant TFI activity appeared in the flow through. The column was then wased with 70 ml of PNBT and 70 ml of the same solution without Triton X-100. The bound TFI were eluted with 0.1 M glycine/HCl, pH 2.2, and concentrated to approximately 0.6 ml by vacuum dialysis against 0.1 M glycine/HCl, pH 2.2.

## Results:

By immunoaffinity chromatography on an anti-TFI-peptide-Ig Sepharose 4B column, TFI was isolated from a number of liver derived cell lines, Chang liver, SK hepatoma and HepG2 hepatoma. Figure 12A shows the SDS-PAGE of the proteins eluted from the anti-TFI-peptide-Ig Sepharose column. In different preparations, one observes somewhat different protein profiles. In some preparations, a 40 kDa protein is the only major protein (lanes 4 and 5); in others, a number of protein bands coexist and a 38 kDa protein is a prominent band instead of the 40 kDa protein. To establish which proteins are TFI related, the $^{125}I$-$X_a$ binding study was performed. The isolated TFI samples were electrophoresed in a 12% SDS-polyacrylamide gel and the proteins were then electrophoretically transblotted onto a nitrocellulose paper and screened for $^{125}I$-$X_a$ binding activity. It was found that three major bands with apparent molecular weights of 40, 38, and 25 kDa possess $^{125}I$-$X_a$ binding activity while other bands are not. (Figure 12B). Sequence analysis of the 38 kDa band from SK hepatoma cells shows that it possesses the same amino terminal sequence as the 40 kDa TFI isolated from HepG2 cells in Example 1, above. Based on the immunoaffinity, amino acid sequencing, and the $^{125}I$-$X_a$ binding studies, it appears that the 40, 38, and 25 kDa inhibitors may be derived from the same molecule.

The results of the above laboratory preparative work leading to the isolation of TFI from several cell sources by immunoaffinity chromatography are further exemplified by the following detailed description of FIGS. 12(A) and 12(B) of the drawings.

FIG. 12. This figure shows the SDS-PAGE of immunoaffinity isolated TFI and screening of $^{125}I$-$X_a$ binding activity. (A) SDS-PAGE. Electrophoresis was carried out on a 12% polyacrylamide gel. Lane 1, molecular weight markers; lane 2, Chang liver TFI; lane 3, SK hepatoma TFI (preparation 1); lane 4, SK hepatoma TFI (preparation 2); lane 5, HepG2 TFI (preparation 1); lane 6, HepG2 TFI (preparation 2). (B) Screening of $^{125}$I-$X_a$ binding activity. Samples were electrophoresed on a 12% polyacrylamide gel. The proteins were electrophoretically transblotted onto a nitrocellulose paper using Bio-Rad Trans-Blot® apparatus and procedure. After the transfer, the nitrocellulose paper was first gently shaken in PBB solution (PBS containing 5 mg/ml BSA and 2.5 mg/ml bovine gamma globulin) and then in PBB solution containing 400 ng/ml $^{125}$-I-$X_a$, each at room temperature for I hour. The nitrocellulose paper was then dried and autoradiographed for 3 days using Kodak XAR-5 film. Lane 1, Chang liver TFI; lane 2, SK hepatoma TFI (preparation-1); lane 3, SK hepatoma TFI (preparation 2); and lane 4, HepG2 TFI (preparation 2).

various other examples will be apparent to the person skilled in the art after reading the present disclosure without departing from the spirit and scope of the present invention. It is intended that all such other examples be included within the scope of the appended claims.

**Claims**

1. Tissue factor inhibitor having the following characteristics:

A. it exists in two forms, $TFI_1$ migrating at about 37-40,000 daltons and $TFI_2$ at about 25-26,000 daltons, as determined by sodium dodecylsulfate polyacrylamide gel electrophoresis,

B. it has a partial N-terminal amino acid sequence as follows:

```
1                                                          15
X-X-Glu-Glu-Asp-Glu-Glu-His-Thr-Ile-Ile-Thr-Asp-Thr-Glu-

16                                          27
Leu-Pro-Pro-Leu-Lys-Leu-Met-His-Ser-Phe-(Phe)-Ala
```

wherein X-X has not been determined, and

C. it exhibits inhibitory activity to Factor $X_a$.

2. Tissue factor inhibitor of Claim 1 isolated from the conditioned medium of HepG2 cells grown in nutrient culture medium.

3. The method of isolating tissue factor inhibitor from the conditioned medium of HepG2 cells comprising:

A. $CdCl_2$ precipitation,

B. Factor $X_a$ Affi-Gel 15 affinity chromatography of the resolubilized precipitate, and

C. Superose 12 gel filtration of the fractions from the affinity chromatography containing TFI activity.

4. The method of isolating tissue factor inhibitor from the conditioned medium of HepG2 cells comprising:

A. $CdCl_2$ precipitation,

B. diisopropylphosphoryl ($ipr_2P$) -Factor $X_a$ Affi-Gel 15 affinity chromatography of the resolubilized precipitate,

C. Sephadex G-75 gel filtration of the fractions from the affinity chromatography containing TFI activity, and

D. Mono Q ion-exchange chromatography of the fractions from the gel filtration containing TFI activity.

5. The method of inhibiting Factor $X_a$ production in a mammal comprising administering to said mammal an effective amount of tissue factor inhibitor as defined in Claim 1.

6. The method of inhibiting Factor $VII_a$/TF enzymatic complex formation in a mammal comprising administering to said mammal an effective amount of tissue factor inhibitor as defined in Claim 1.

FIG. 1.

FIG. 2.

FIG. 3.

FIG. 4.

0300988

FIG.5.

FIG.6.

0300988

FIG.7.

FIG.8.

FIG.9.

FIG.10.

FIG. 11.

FIG. 12.